# EUROPEAN PATENT SPECIFICATION

(11) **EP 4 302 619 B1**
(45) Date of publication and mention of the grant of the patent: **28.01.2026**
(21) Application number: 23178990.0
(22) Date of filing: 13.06.2023
(51) Int. Cl.: A24D 1/02, A24D 1/20

(54) **AEROSOL-GENERATING SUBSTRATE**
AEROSOLERZEUGENDES SUBSTRAT
SUBSTRAT DE GÉNÉRATION D'AÉROSOL

(30) Priority: 14.06.2022 CN 202210666979
(43) Date of publication of application: 10.01.2024
(73) Proprietor: Shenzhen Smoore Technology Limited, Shenzhen, Guangdong 518102 (CN)
(72) Inventor: WEN, Zhihua, Shenzhen, 518102 (CN); ZHANG, Dazhi, Shenzhen, 518102 (CN)
(74) Representative: Corradini, Corrado

(56) References cited:
- WO-A1-2021/180967
- CN-A- 112 754 067
- KR-A- 20200 061 290
- KR-U- 20120 000 088

## Description

### TECHNICAL FIELD

The present application relates to the technical field of atomization, and particularly to aerosol-generating substrates.

### BACKGROUND

An aerosol is a colloidal dispersion system formed by dispersing and suspending small solid or liquid particles in a gas medium. The aerosol can be absorbed by the human body through the respiratory system, providing users with a new alternative absorption method. An atomizer refers to a device that is adapted to form an aerosol from a stored atomizable substrate by means of heating or ultrasound treatment. Atomizable substrates may include e-liquids containing nicotine, medical drugs, skin care lotions, etc. The atomization of these substrates can deliver inhalable aerosols to the users, replacing conventional product forms and absorption methods.

A heat-not-burn aerosol-generating substrate is a new kind of substrate that is to be heated by a special heat source and substrate (the heating temperature is below 500°C or even lower), and various substances in the substrate atomized in heating are volatilized to produce an aerosol to meet the needs of smokers. The heat-not-burn aerosol-generating substrate does not produce flames and soot during the suction process, which is friendly to the environment and can provide the users with a good experience while reducing harmful substances that are produced by pyrolysis of conventional substrates during combustion, and thus is less harmful to the users.

However, due to a structural defect of the atomizer, a part of the aerosol generated by heating and volatilizing an existing heat-not-burn atomizable substrate cannot flow out smoothly with the airflow, so that effective substances of the heat-not-burn atomizable substrate, such as nicotine, cannot be well released, which affects the further improvement of user experience. Related technologies are known from KR20120000088U, CN112754067A, WO2021180967A1, and KR20200061290A.

### SUMMARY

In view of this, aiming to address the problem that the aerosol cannot flow out smoothly, there is a need to provide an aerosol-generating substrate, and the aerosol-generating substrate can allow the aerosol to flow out smoothly. The present invention is set out in the appended claims. Further aspects and preferred embodiments are defined in the dependent claims. Aspects, embodiments, examples, and implementations of the present disclosure that do not fall within the scope of the appended claims do not form part of the invention and are merely provided for illustrative purposes.

In the aerosol-generating substrate, the amount of air entered the confluence sub-section and the total ventilation rate can be effectively increased by arranging the air inlet for substrate section in the first wrapping layer at a distance of less than 1 mm from the end surface of the confluence and filtration section. When a negative pressure is generated at the end surface of the aerosol-generating substrate away from the substrate section by suction of a user, nicotine and flavor substances generated by the substrate body during the heating process is easier to be gradually drawn out, thereby providing a better suction consistency and effectively improving the user experience. Moreover, since the position of the air inlet for substrate section is very close to that of the end surface of the substrate section, the amount of air passing through the substrate body can be minimized, so that the substrate body can be in a negative-pressure oxygen-poor state to the greatest extent. Thus, the effective substances in the aerosol-generating substrate can be better released, and during the dynamic suction process, the temperature of the substrate body can be relatively stable, so that the release amount of the aerosol can remain consistent.

The above description is only a summary of the technical solution of the present application. In order to better understand the technical means of the present application and to implement according to the contents of the description, and in order to make the above and other purposes, features, and advantages of the present application more clear and understandable, the specific implementations of the present application are specifically described as follows.

### BRIEF DESCRIPTION OF THE DRAWINGS

Various other advantages and benefits will become apparent to those of ordinary skill in the art upon reading the following detailed description of the preferred embodiments. The drawings are only for the purpose of illustrating the preferred embodiments and are not to be considered as limitations of the present application. The same reference signs are used to denote the same components throughout the drawings.
FIG. 1 is a schematic view of an embodiment of an aerosol-generating substrate of the present application.
FIG. 2 is a schematic view of an internal structure of the aerosol-generating substrate shown in FIG. 1.

### Reference signs:

100, aerosol-generating substrate; 120, substrate section; 121, substrate body; 123, first wrapping layer; 123a, air inlet for substrate section; 140, confluence and filtration section; 141, confluence sub-section; 1412, hollow tube; 1414, second wrapping layer; 1414a, cavity; 1414b, air inlet for confluence sub-section; 143, filtration sub-section; 1432, filter medium; 1434, third wrapping layer.

### DETAILED DESCRIPTION

In order to make the above objectives, features and advantages of the present application more clear and understandable, embodiments of the present disclosure will be described in detail below with reference to the accompanying drawings. In the following description, many specific details are described to make the present disclosure fully understandable. However, the present disclosure can be implemented in many other ways different from those described herein. Therefore, the present disclosure is not limited by the specific embodiments disclosed below.

In the description of the present disclosure, it should be understood that the terms "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial" , "radial", "circumferential", etc. indicate the orientations or positional relationships on the basis of the drawings. These terms are only for describing the present disclosure and simplifying the description, rather than indicating or implying that the related devices or elements must have the specific orientations, or be constructed or operated in the specific orientations, and therefore cannot be understood as limitations of the present disclosure.

In addition, the terms "first" and "second" are used merely as labels to distinguish one element having a certain name from another element having the same name, and cannot be understood as indicating or implying any priority, precedence, or order of one element over another, or indicating the quantity of the element. Therefore, the element modified by "first" or "second" may explicitly or implicitly includes at least one of the elements. In the description of the present disclosure, "a plurality of" means at least two, such as two, three, etc., unless otherwise specifically defined.

In the present disclosure, unless otherwise clearly specified and defined, the terms "installed", "connected", "coupled", "fixed" and other terms should be interpreted broadly. For example, an element, when being referred to as being "installed", "connected", "coupled", "fixed" to another element, unless otherwise specifically defined, may be fixedly connected, detachably connected, or integrated to the other element, may be mechanically connected or electrically connected to the other element, and may be directly connected to the other element or connected to the other element via an intermediate element. For those of ordinary skill in the art, the specific meaning of the above-mentioned terms in the present disclosure can be understood according to specific circumstances.

In the present disclosure, unless otherwise specifically defined, an element, when being referred to as being located "on" or "under" another element, may be in direct contact with the other element or contact the other element via an intermediate element. Moreover, the element, when being referred to as being located "on", "above", "over" another element, may be located right above or obliquely above the other element, or merely located at a horizontal level higher than the other element; the element, when being referred to as being located "under", "below", "beneath" another element, may be located right below or obliquely below the other element, or merely located at a horizontal level lower than the other element.

It should be noted that an element, when being referred to as being "fixed" or "mounted" to another element, may be directly fixed or mounted to the other element or via an intermediate element. Such terms as "vertical", "horizontal", "up", "down", "left", "right" and the like used herein are for illustrative purposes only and are not meant to be the only ways for implementing the present disclosure.

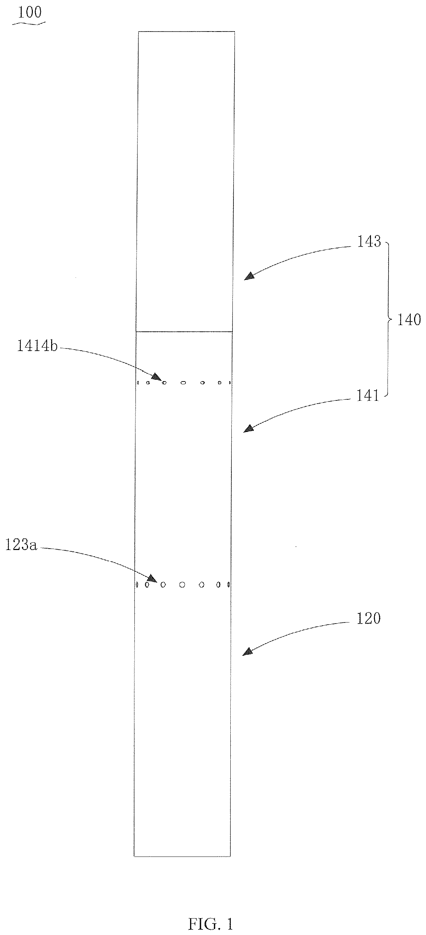

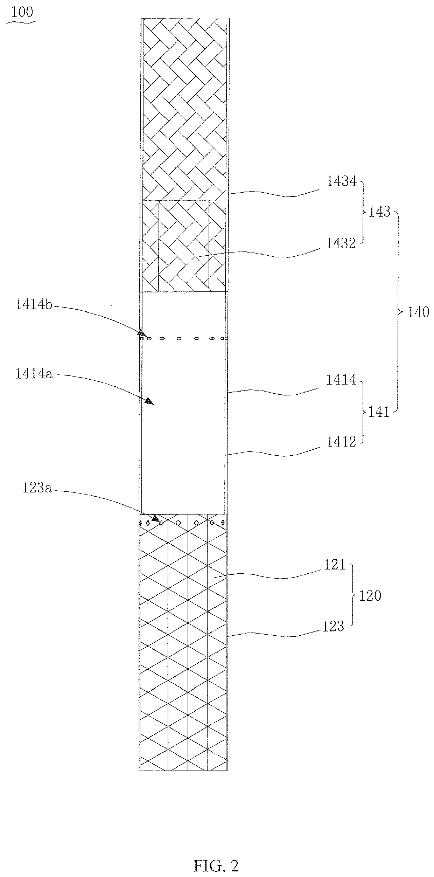

Referring to FIG. 1 and FIG. 2, FIG. 1 is a schematic view of an embodiment of an aerosol-generating substrate of the present application, and FIG. 2 is a schematic view of an internal structure of the aerosol-generating substrate shown in FIG. 1.

An embodiment of the present application provides an aerosol-generating substrate 100. The aerosol-generating substrate 100 has a columnar structure with a round cross-section. The aerosol-generating substrate 100 is adapted to be inserted into a heating air channel of an atomizer. The atomizer is adapted to heat the aerosol-generating substrate 100 to generate an aerosol for a user to inhale.

The aerosol-generating substrate 100 has the columnar structure as a whole, and the cross-section of the aerosol-generating substrate 100 is round. The aerosol-generating substrate 100 includes a substrate section 120 and a confluence and filtration section 140 connected with each other. The confluence and filtration section 140 is connected to one end of the substrate section 120 in the axial direction of the substrate section. The substrate section 120 can generate an aerosol under the heating of the atomizer. The aerosol is filtered by the confluence and filtration section 140, and then flows out from the aerosol-generating substrate 100 for the user to inhale. It can be understood that the shape and size of the aerosol-generating substrate 100 are not limited, and the cross-section of the aerosol-generating substrate 100 can have a regular or irregular shape such as an elliptical or a rectangular shape.

The substrate section 120 includes a substrate body 121 and a wrapping layer for substrate section. The wrapping layer for substrate section is wrapped around the substrate body 121 to form a columnar structure. The outer surface of the wrapping layer for substrate section forms the columnar outer surface of the columnar structure. The cross-section of the columnar structure perpendicular to the axial direction can be a round shape. It can be understood that the shape of the cross-section of the columnar structure is not limited, and in some other embodiments, can be a regular or irregular shape such as an elliptical or a rectangular shape.

Further, the substrate body 121 can include tobacco fillers. The tobacco fillers can be in regular or irregular shapes such as filaments, granules, or sheets. It can be understood that the material forming the substrate body 121 is not limited. The substrate body 121 can be made of a single material or mixed multiple materials in different proportions. The substrate body 121 can further include other substances, so as to produce an aerosol with different components and different tastes to meet various needs of users. The wrapping layer for substrate section can be made of an overwrap material, such as cigarette paper, so as to keep the substrate body 121 in a certain shape. It can be understood that the material of the wrapping layer for substrate section is not limited, and in some other embodiments, the wrapping layer for substrate section can be made of another material, such as aluminum foil to meet different requirements.

Referring still to FIG. 1 and FIG. 2, the confluence and filtration section 140 includes a confluence sub-section 141 and a filtration sub-section 143. The confluence sub-section 141 is connected to one end of the substrate section 120 in the axial direction of the substrate section 120, and the filtration sub-section 143 is connected to the other end of the confluence sub-section 141 in the axial direction, and the other end of the confluence sub-section 141 is away from the substrate section 120. In this way, the aerosol flows generated by heating the substrate section 120 can flow out after being merged in the confluence sub-section 141 and filtered in the filtration sub-section 143.

In some embodiments, the confluence sub-section 141 includes a hollow tube 1412 and a wrapping layer for confluence sub-section. The hollow tube 1412 is a hollow tubular structure with openings at both ends. The wrapping layer for confluence sub-section can be made of an overwrap material such as molding paper. The wrapping layer for confluence sub-section is wrapped around the outer columnar surface of the hollow tube 1412 in the circumferential direction to form an integrated structure. In this way, a cavity 1414a that is in communication with the substrate section 120 is defined in the confluence sub-section 141. The aerosol flowing out from the substrate section 120 enters the cavity 1414a and merges with the airflow in the cavity 1414a, and then is entrained in the airflow and flows into the filtration sub-section 143. It can be understood that the shape and structure of the confluence sub-section 141 are not limited. The cross-section of the confluence sub-section 141 can have a round, square or other regular or irregular shape, and the materials forming the hollow tube 1412 and the wrapping layer for confluence sub-section can be selected as needed to meet different requirements.

In some embodiments, the filtration sub-section 143 includes a filter medium 1432 and a filtration sub-section wrapping layer. The filtration sub-section wrapping layer is wrapped around the filter medium 1432 to form a columnar structure. The outer surface of the filtration sub-section wrapping layer forms the outer columnar surface of the columnar structure, and the cross-section of the columnar structure perpendicular to the axial direction has a round shape. It can be understood that the shape of the cross-section of the columnar structure is not limited, and in some other embodiments, can be a regular or irregular shape such as an elliptical or a rectangular shape.

The filter medium 1432 is made of a polymer filler material, which can filter and cool down the aerosol flowing therethrough. The filtration sub-section wrapping layer can be made of an overwrap material, such as molding paper. It can be understood that the materials forming the filter medium 1432 and the filtration sub-section wrapping layer are not limited, and in other embodiments, the filter medium 1432 and the filtration sub-section wrapping layer can be made of other materials to meet different filtration requirements.

The aerosol-generating substrate 100 further includes a wrapping layer for confluence and filtration section and a connecting layer. The wrapping layer for confluence and filtration section is made of an overwrap material, such as molding paper. The wrapping layer for confluence and filtration section is wrapped around the outer surface of the wrapping layer for confluence sub-section and the filtration sub-section wrapping layer to form an integrated confluence and filtration section 140. The connecting layer is made of an overwrap material, such as tipping paper. The connecting layer is wrapped around one end of the wrapping layer for confluence and filtration section adjacent to the substrate section 120 and one end of the wrapping layer for substrate section adjacent to the confluence sub-section 141 to form an integrated aerosol-generating substrate 100. It can be understood that the materials forming the wrapping layer for confluence and filtration section and the connecting layer are not limited, and can be selected as needed to meet different requirements.

In this way, the substrate section 120 includes the substrate body 121 and a first wrapping layer 123 circumferentially wrapping around the substrate body 121. The first wrapping layer 123 is a layer stack composed by the wrapping layer for substrate section wrapping around the substrate body 121 and the connecting layer stacked on and wrapping around the end of the wrapping layer for substrate section adjacent to the confluence sub-section 141. The confluence sub-section 141 includes the hollow tube 1412 and a second wrapping layer 1414 circumferentially wrapping around the outer surface of the hollow tube 1412. The second wrapping layer 1414 is a layer stack composed by the wrapping layer for confluence sub-section wrapping around the hollow tube 1412 and the wrapping layer for confluence and filtration section wrapping around the wrapping layer for confluence sub-section. The filtration sub-section 143 includes the filter medium 1432 and a third wrapping layer 1434 circumferentially wrapping around the filter medium 1432. The third wrapping layer 1434 is a layer stack composed by the filtration sub-section wrapping layer wrapping around the filter medium 1432 and the wrapping layer for confluence and filtration section wrapping around the filtration sub-section wrapping layer.

The first wrapping layer 123 is defined as a general term for the stacked layer structure wrapping around the substrate body 121. The second wrapping layer 1414 is defined as a general term for the stacked layer structure wrapping around the outer columnar surface of the hollow tube 1412. The third wrapping layer 1434 is defined as a general term for the stacked layer structure wrapping around the filter medium 1432. The specific structures and materials of the first wrapping layer 123, the second wrapping layer 1414, and the third wrapping layer 1434 are not limited, and can be selected as needed to meet different requirements.

In the present application, the first wrapping layer 123 wrapping around the substrate body 121 is provided with an air inlet for substrate section 123a that connects the inner surface and the outer surface of the first wrapping layer 123. A distance between the air inlet 123a for substrate section and an end surface of the substrate section 120 connected to the confluence and filtration section 140 is greater than 0 and smaller than 1 mm. Specifically, since the first wrapping layer 123 is formed by the wrapping layer for substrate section wrapping around the substrate body 121 and the connecting layer stacked on and wrapping around the end of the wrapping layer for substrate section adjacent to the confluence sub-section 141, the air inlet 123a for substrate section extends in the thickness direction through the wrapping layer for substrate section and the connecting layer.

In this way, by arranging the air inlet 123a for substrate section in the first wrapping layer 123 at a distance of less than 1 mm from the end surface of the confluence and filtration section 140, the amount of air entered the confluence sub-section 141 and the total ventilation rate can be effectively increased. When a negative pressure is generated at the end surface of the aerosol-generating substrate 100 away from the substrate section 120 by suction of the user, nicotine and flavor substances generated by the substrate body 121 during the heating process is easier to be gradually drawn out, thereby providing a better suction consistency and effectively improving the user experience. Moreover, since the position of the air inlet 123a for substrate section is very close to that of the end surface of the substrate section 120, the amount of air passing through the substrate body 121 can be minimized, so that the substrate body 121 can be in a negative-pressure oxygen-poor state to the greatest extent. Thus, the effective substances in the aerosol-generating substrate can be better released, and during the dynamic suction process, the temperature of the substrate body 121 can be relatively stable, so that the release amount of the aerosol can remain consistent.

The substrate section 120 is provided with a plurality of air inlets 123a for substrate section, and the air inlets 123a for substrate section are distributed at intervals in the circumferential direction of the confluence and filtration section 140. In this way, the airflow outside the aerosol-generating substrate 100 can enter the substrate section 120 separately through the plurality of air inlets 123a for substrate section. As a preferred embodiment, the number of the air inlets 123a for substrate section is 2 to 20. It can be understood that the number of the air inlets 123a for substrate section is not limited, and can be set as needed to meet different suction requirements. For example, the number of the air inlets 123a for substrate section is 16 or 12.

Furthermore, according to the difference in the amount of airflow in different regions, the air inlets 123a for substrate section are distributed non-equidistantly in the circumferential direction of the substrate section 120, that is, the distance between two adjacent air inlets 123a for substrate section is different from the distance between other two adjacent air inlets 123a for substrate section. As compared with the equidistant distribution, the non-equidistant distribution of the air inlets 123a for substrate section can make the taste of the aerosol different, thereby diversifying the aerosol taste. In addition, the air inlets 123a for substrate section can be distributed corresponding to the heaters of the atomizer which may be non-equidistantly distributed in the heating air channel. By distributing the air inlets 123a for substrate section corresponding to the heaters, the aerosol-generating substance can be more uniformly heated and effectively atomized. It can be understood that, in some other embodiments, all the air inlets 123a for substrate section can be distributed equidistantly along the circumferential direction of the substrate section 120, that is, the air inlets 123a for substrate section are at equal intervals.

The confluence sub-section 141 is provided with at least one set of air inlets 1414b for confluence sub-section. The cavity 1414a is in communication with external atmosphere through each set of air inlets 1414b for confluence sub-section. Since the confluence sub-section 141 includes the hollow tube 1412 and the second wrapping layer 1414 circumferentially wrapping around the outer surface of the hollow tube 1412, and the second wrapping layer 1414 is formed by the wrapping layer for confluence sub-section wrapping around the hollow tube 1412 and the wrapping layer for confluence and filtration section stacked on and wrapping around the wrapping layer for confluence sub-section, the air inlets 1414b for confluence sub-section extend through the hollow tube 1412, the wrapping layer for confluence sub-section, and the wrapping layer for confluence and filtration section. In this way, the airflow outside the aerosol-generating substrate 100 can enter the cavity 1414a of the confluence sub-section 141 through the air inlets 1414b for confluence sub-section, thereby further increasing the air intake and the total ventilation rate of the aerosol-generating substrate 100.

The distance between the air inlets 1414b for confluence sub-section and the filtration sub-section 143 is 1 mm to 10 mm. It can be understood that the distance between the air inlets 1414b for confluence sub-section and the filtration sub-section 143 can be set as needed to meet different requirements.

Specifically, in some embodiments, the confluence sub-section 141 is provided with multiple sets of air inlets 1414b for confluence sub-section, and the multiple sets of air inlets 1414b for confluence sub-section are distributed at intervals along the axial direction of the confluence sub-section 141. Each set of air inlets 1414b for confluence sub-section includes a plurality of air inlets 1414b for confluence sub-section, and all of the air inlets 1414b for confluence sub-section in the same set are distributed at intervals along the circumferential direction of the confluence sub-section 141. The distances between adjacent sets of air inlets 1414b for confluence sub-section can be the same or different. The air inlets 1414b for confluence sub-section in two adjacent sets can be correspondingly distributed in a one-to-one manner or be staggered with each other in the axial direction of the confluence sub-section 141.

Preferably, the confluence sub-section 141 is provided with one, two or three sets of air inlets 1414b for confluence sub-section, and the number of the air inlets 1414b for confluence sub-section in each set is 2 to 20. It can be understood that the number of the sets of air inlets 1414b for confluence sub-section and the number of the air inlets 1414b for confluence sub-section in each set are not limited, and can be set as needed to meet different requirements. The numbers of the air inlets 1414b for confluence sub-section in different sets can be the same or different. For example, the number of the air inlets 1414b for confluence sub-section in each set is 16 or 12.

Further, in some embodiments, all the air inlets 1414b for confluence sub-section in the same set are distributed equidistantly along the circumferential direction of the confluence sub-section 141, that is, the air inlets 1414b for confluence sub-section are at equal intervals. The airflow can uniformly flow into the confluence sub-section 141 through the equidistantly distributed air inlets 1414b for confluence sub-section, thereby homogenizing the aerosol generated from the substrate section 120. In some other embodiments, according to the difference in the amount of airflow in different regions, the air inlets 1414b for confluence sub-section in the same set are distributed non-equidistantly in the circumferential direction of the confluence sub-section 141, that is, the distance between two adjacent air inlets 1414b for confluence sub-section is different from the distance between other two adjacent air inlets 1414b for confluence sub-section. The airflow non-uniformly flows into the confluence sub-section 141 through the non-equidistantly distributed air inlets 1414b for confluence sub-section, thereby allowing the aerosol generated from the substrate section 120 to have a different taste.

The air intake and the total ventilation rate of the aerosol-generating substrate 100 can be significantly improved by providing the air inlet 123a for substrate section in the substrate section 120 at a distance greater than 0 and smaller than 1 mm from the end surface of the substrate section 120 adjacent to the filter section 140. Since the air inlet 123a for substrate section is close enough to the filter section 140, in the process of heating the aerosol-generating substrate 100, nicotine and flavor substances produced by the substrate body 121 can be fully extracted and drawn out with the airflow, thereby improving the user experience. Moreover, the airflow outside the aerosol-generating substrate 100 can also enter the cavity 1414a of the confluence sub-section 141 through the air inlets 1414b for confluence sub-section, thereby further increasing the air intake and the total ventilation rate of the aerosol-generating substrate 100.

## Claims

1. An aerosol-generating substrate, comprising:
a substrate section (120) comprising a substrate body (121) and a first wrapping layer (123) circumferentially wrapping around the substrate body (121);
a confluence and filtration section (140) connected to one end of the substrate section (120) in the axial direction of the substrate section to filter the airflow flowing out from the substrate section (120);
wherein the first wrapping layer (123) is provided with a plurality of air inlets (123a) for substrate section (120), the air inlets (123a) for substrate section (120) are distributed at intervals in the circumferential direction of the substrate section (120), and the air inlets (123a) for substrate section (120) connects the inner surface and the outer surface of the first wrapping layer (123);
wherein the confluence and filtration section (140) comprises:
a confluence sub-section (141) connected to the end of the substrate section (120) in the axial direction, the confluence sub-section (141) defining a cavity (1414a) allowing airflow to flow therethrough; and
a filtration sub-section (143) connected to the end of the confluence sub-section (141) away from the substrate section (120), to filter airflow flowing out from the cavity (1414a);
wherein the confluence sub-section (141) is provided with at least one set of air inlets (1414b) for confluence sub-section, the cavity (1414a) is in communication with external atmosphere through each set of air inlets (1414b) for confluence sub-section;
**characterized in that**
the distance between any set of air inlets (1414b) for confluence sub-section and the filtration sub-section (143) is 1 mm to 10 mm; and
the distance between the plurality of air inlets (123a) for substrate section (120) and the surface of the end of the substrate section (120) connected to the confluence and filtration section (140) is greater than 0 mm and smaller than 1 mm, while the air inlets (123a) for substrate section (120) are distributed non-equidistantly in the circumferential direction of the substrate section (120).

2. The aerosol-generating substrate according to claim 1, wherein the number of the air inlets (123a) for substrate section (120) is 2 to 20.

3. The aerosol-generating substrate according to claim 1, wherein the confluence sub-section (141) is provided with multiple sets of air inlets (1414b) for confluence sub-section, the multiple sets of air inlets (1414b) for confluence sub-section are distributed at intervals along the axial direction of the confluence sub-section (141), each set of air inlets (1414b) for confluence sub-section comprises a plurality of air inlets (1414b) for confluence sub-section, and the plurality of air inlets (1414b) for confluence sub-section in each set of the air inlets (1414b) for confluence sub-section are distributed at intervals along the circumferential direction of the confluence sub-section (141).

4. The aerosol-generating substrate according to claim 3, wherein the plurality of air inlets (1414b) for confluence sub-section in each set of the air inlets (1414b) for confluence sub-section are equidistantly distributed along the circumferential direction of the confluence sub-section (141).

5. The aerosol-generating substrate according to claim 3, wherein the number of the air inlets (1414b) for confluence sub-section in each set of air inlets for confluence sub-section is 2 to 20.

6. The aerosol-generating substrate according to any one of claims 1 to 5, wherein the first wrapping layer (123) comprising a wrapping layer for substrate section (120) wrapping around the substrate body (121) and a connecting layer stacked on and wrapping around the end of the wrapping layer for substrate section (120) adjacent to the confluence sub-section (141).

7. The aerosol-generating substrate according to any one of claims 1 to 6, wherein the confluence sub-section (141) comprises a hollow tube (1412) and a second wrapping layer (1414) circumferentially wrapping around the outer surface of the hollow tube (1412).

8. The aerosol-generating substrate according to claim 7, wherein the second wrapping layer (1414) comprises a wrapping layer for confluence sub-section wrapping around the hollow tube (1412) and a wrapping layer for confluence and filtration section (140) wrapping around the confluence sub-section wrapping layer.

9. The aerosol-generating substrate according to any one of claims 1 to 8, wherein the filtration sub-section (143) comprises a filter medium (1432) and a third wrapping layer (1434) circumferentially wrapping around the filter medium (1432).

10. A system comprising an atomizer and the aerosol-generating substrate according to any one of claims 1 to 9, wherein the atomizer comprising a heating air channel, and the aerosol-generating substrate is in the heating air channel.

## Patentansprüche

1. Aerosol-erzeugendes Substrat, umfassend:
einen Substratabschnitt (120), umfassend einen Substratkörper (121) und eine erste Umhüllungsschicht (123), die den Substratkörper (121) in Umfangsrichtung umhüllt;
einen Zustrom- und Filtrationsabschnitt (140), der in axialer Richtung des Substratabschnitts mit einem Ende des Substratabschnitts (120) verbunden ist, um den Luftstrom zu filtern, der aus dem Substratabschnitt (120) ausströmt;
wobei die erste Umhüllungsschicht (123) mit einer Vielzahl von Lufteinlässen (123a) für Substratabschnitt (120) versehen ist, die Lufteinlässe (123a) für Substratabschnitt (120) in Abständen in der Umfangsrichtung des Substratabschnitts (120) verteilt sind, und die Lufteinlässe (123a) für Substratabschnitt (120) die Innenfläche und die Außenfläche der ersten Umhüllungsschicht (123) verbinden;
wobei der Zustrom- und Filtrationsabschnitt (140) Folgendes umfasst:
einen Zustromunterabschnitt (141), der in der axialen Richtung mit dem Ende des Substratabschnitts (120) verbunden ist, wobei der Zustromunterabschnitt (141) einen Hohlraum (1414a) definiert, der ermöglicht, dass ein Luftstrom dort hindurch strömt; und einen Filtrationsunterabschnitt (143), der mit dem vom Substratabschnitt (120) entfernten Ende des Zustromunterabschnitts (141) verbunden ist, um einen Luftstrom zu filtern, der aus dem Hohlraum (1414a) ausströmt;
wobei der Zustromunterabschnitt (141) mit mindestens einem Satz von Lufteinlässen (1414b) für den Zustromunterabschnitt versehen ist, der Hohlraum (1414a) durch jeden Satz von Lufteinlässen (1414b) für den Zustromunterabschnitt mit der äußeren Atmosphäre in Verbindung ist;
**dadurch gekennzeichnet, dass**
der Abstand zwischen einem beliebigen Satz von Lufteinlässen (1414b) für Zustromunterabschnitt und dem Filtrationsunterabschnitt (143) 1 mm bis 10 mm ist; und der Abstand zwischen der Vielzahl von Lufteinlässen (123a) für Substratabschnitt (120) und der Oberfläche des Endes des Substratabschnitts (120), das mit dem Zustrom- und Filtrationsabschnitt (140) verbunden ist, größer als 0 mm und kleiner als 1 mm ist, während die Lufteinlässe (123a) für Substratabschnitt (120) nicht-äquidistant in der Umfangsrichtung des Substratabschnitts (120) verteilt sind.

2. Aerosol-erzeugendes Substrat nach Anspruch 1, wobei die Anzahl der Lufteinlässe (123a) für Substratabschnitt (120) 2 bis 20 ist.

3. Aerosol-erzeugendes Substrat nach Anspruch 1, wobei der Zustromunterabschnitt (141) mit mehreren Sätzen von Lufteinlässen (1414b) für Zustromunterabschnitt versehen ist, die mehreren Sätze von Lufteinlässen (1414b) für Zustromunterabschnitt in Abständen entlang der axialen Richtung des Zustromunterabschnitts (141) verteilt sind, jeder Satz von Lufteinlässen (1414b) für Zustromunterabschnitt eine Vielzahl von Lufteinlässen (1414b) für Zustromunterabschnitt umfasst, und die Vielzahl von Lufteinlässen (1414b) für Zustromunterabschnitt in jedem Satz der Lufteinlässe (1414b) für Zustromunterabschnitt in Abständen entlang der Umfangsrichtung des Zustromunterabschnitts (141) verteilt sind.

4. Aerosol-erzeugendes Substrat nach Anspruch 3, wobei die Vielzahl von Lufteinlässen (1414b) für Zustromunterabschnitt in jedem Satz der Lufteinlässe (1414b) für Zustromunterabschnitt äquidistant entlang der Umfangsrichtung des Zustromunterabschnitts (141) verteilt sind.

5. Aerosol-erzeugendes Substrat nach Anspruch 3, wobei die Anzahl der Lufteinlässe (1414b) für Zustromunterabschnitt in jedem Satz von Lufteinlässen für Zustromunterabschnitt 2 bis 20 ist.

6. Aerosol-erzeugendes Substrat nach einem der Ansprüche 1 bis 5, die erste Umhüllungsschicht (123) umfassend eine Umhüllungsschicht für Substratabschnitt (120), die um den Substratkörper (121) gewickelt ist, und eine Verbindungsschicht, die auf das Ende der Umhüllungsschicht für Substratabschnitt (120) angrenzend an den Zustromunterabschnitt (141) gestapelt ist und dieses umhüllt.

7. Aerosol-erzeugendes Substrat nach einem der Ansprüche 1 bis 6, wobei der Zustromunterabschnitt (141) ein hohles Rohr (1412) und eine zweite Umhüllungsschicht (1414) umfasst, die die Außenfläche des hohlen Rohrs (1412) in Umfangsrichtung umhüllt.

8. Aerosol-erzeugendes Substrat nach Anspruch 7, wobei die zweite Umhüllungsschicht (1414) eine Umhüllungsschicht für Zustromunterabschnitt, die das hohle Rohr (1412) umhüllt, und eine Umhüllungsschicht für Zustroms- und Filtrationsabschnitt (140), die die Zustromunterabschnitt-Umhüllungsschicht umhüllt.

9. Aerosol-erzeugendes Substrat nach einem der Ansprüche 1 bis 8, wobei der Filtrationsunterabschnitt (143) ein Filtermedium (1432) und eine dritte Umhüllungsschicht (1434) umfasst, die das Filtermedium (1432) in Umfangsrichtung umhüllt.

10. System, umfassend einen Zerstäuber und das Aerosol-erzeugende Substrat nach einem der Ansprüche 1 bis 9, wobei der Zerstäuber einen Heizluftkanal und umfasst, und das Aerosol-erzeugende Substrat in dem Heizluftkanal ist.

## Revendications

1. Substrat générateur d'aérosol, comprenant :
une section de substrat (120) comprenant un corps de substrat (121) et une première couche d'enveloppement (123) enveloppant de manière circulaire le corps de substrat (121) ;
une section de confluence et de filtration (140) connectée à une extrémité de la section de substrat (120) dans la direction axiale de la section de substrat afin de filtrer le flux d'air s'écoulant hors de la section de substrat (120) ;
dans lequel la première couche d'enveloppement (123) est pourvue d'une pluralité d'entrées d'air (123a) pour la section de substrat (120), les entrées d'air (123a) pour la section de substrat (120) sont réparties à intervalles réguliers dans la direction circonférentielle de la section de substrat (120), et les entrées d'air (123a) pour la section de substrat (120) connectent la surface intérieure et la surface extérieure de la première couche d'enveloppement (123) ;
dans lequel la section de confluence et de filtration (140) comprend :
une sous-section de confluence (141) connectée à l'extrémité de la section de substrat (120) dans la direction axiale, la sous-section de confluence (141) définissant une cavité (1414a) permettant à l'air de circuler à travers celle-ci ; et
une sous-section de filtration (143) connectée à l'extrémité de la sous-section de confluence (141) éloignée de la section de substrat (120), afin de filtrer le flux d'air sortant de la cavité (1414a) ;
dans lequel la sous-section de confluence (141) est pourvue d'au moins un ensemble d'entrées d'air (1414b) pour la sous-section de confluence, la cavité (1414a) communique avec l'atmosphère extérieure par l'intermédiaire de chaque ensemble d'entrées d'air (1414b) pour la sous-section de confluence ;
**caractérisé en ce que**
la distance entre un quelconque ensemble d'entrées d'air (1414b) pour la sous-section de confluence et la sous-section de filtration (143) est comprise entre 1 mm et 10 mm ; et
la distance entre la pluralité d'entrées d'air (123a) pour la section de substrat (120) et la surface de l'extrémité de la section de substrat (120) connectée à la section de confluence et de filtration (140) est supérieure à 0 mm et inférieure à 1 mm, tandis que les entrées d'air (123a) pour la section de substrat (120) sont réparties de manière non équidistante dans la direction circonférentielle de la section de substrat (120).

2. Substrat générateur d'aérosol selon la revendication 1, dans lequel le nombre d'entrées d'air (123a) pour la section de substrat (120) est compris entre 2 et 20.

3. Substrat générateur d'aérosol selon la revendication 1, dans lequel la sous-section de confluence (141) est pourvue de plusieurs ensembles d'entrées d'air (1414b) pour la sous-section de confluence, les ensembles d'entrées d'air (1414b) pour la sous-section de confluence sont répartis à intervalles réguliers le long de la direction axiale de la sous-section de confluence (141), chaque ensemble d'entrées d'air (1414b) pour la sous-section de confluence comprend une pluralité d'entrées d'air (1414b) pour la sous-section de confluence, et la pluralité d'entrées d'air (1414b) pour la sous-section de confluence dans chaque ensemble d'entrées d'air (1414b) pour la sous-section de confluence sont réparties à intervalles réguliers le long de la direction circonférentielle de la sous-section de confluence (141).

4. Substrat générateur d'aérosol selon la revendication 3, dans lequel la pluralité d'entrées d'air (1414b) pour la sous-section de confluence dans chaque ensemble d'entrées d'air (1414b) pour la sous-section de confluence sont réparties de manière équidistante le long de la direction circonférentielle de la sous-section de confluence (141).

5. Substrat générateur d'aérosol selon la revendication 3, dans lequel le nombre d'entrées d'air (1414b) pour la sous-section de confluence dans chaque ensemble d'entrées d'air pour la sous-section de confluence est compris entre 2 et 20.

6. Substrat générateur d'aérosol selon l'une quelconque des revendications 1 à 5, dans lequel la première couche d'enveloppement (123) comprend une couche d'enveloppement pour la section de substrat (120) s'enroulant autour du corps de substrat (121) et une couche de connexion empilée sur et s'enroulant autour de l'extrémité de la couche d'enveloppement pour la section de substrat (120) adjacente à la sous-section de confluence (141).

7. Substrat générateur d'aérosol selon l'une quelconque des revendications 1 à 6, dans lequel la sous-section de confluence (141) comprend un tube creux (1412) et une deuxième couche d'enveloppement (1414) enveloppant de manière circulaire la surface extérieure du tube creux (1412).

8. Substrat générateur d'aérosol selon la revendication 7, dans lequel la deuxième couche d'enveloppement (1414) comprend une couche d'enveloppement pour la sous-section de confluence s'enroulant autour du tube creux (1412) et une couche d'enveloppement pour la section de confluence et de filtration (140) s'enroulant autour de la couche d'enveloppement de la sous-section de confluence.

9. Substrat générateur d'aérosol selon l'une quelconque des revendications 1 à 8, dans lequel la sous-section de filtration (143) comprend un milieu filtrant (1432) et une troisième couche d'enveloppement (1434) enveloppant de manière circulaire le milieu filtrant (1432).

10. Système comprenant un atomiseur et le substrat générateur d'aérosol selon l'une quelconque des revendications 1 à 9, dans lequel l'atomiseur comprend un canal d'air chauffant, et le substrat générateur d'aérosol se trouve dans le canal d'air chauffant.
